# EUROPEAN PATENT APPLICATION

(11) **EP 3 922 759 A1**
(43) Date of publication of application: **15.12.2021**
(21) Application number: 20305637.9
(22) Date of filing: 11.06.2020
(51) Int. Cl.: C25B 3/02

(54) **PROCESS OF ALPHA,BETA-DESATURATION OF COMPOUNDS CONTAINING A CARBONYL MOIETY**

(71) Applicant: Minakem, 59640 Dunkerque (FR)
(72) Inventor: BARAN, Phil, San Diego, CA 92130 (US); GNAIM, Samer, 3010000 Baqa El-Garbia (IL); ECHEVERRIA, Pierre-Georges, 59800 Lille (FR); PETIT, Laurent, 59800 Lille (FR)
(74) Representative: Plasseraud IP

(57) **Abstract**

The present invention relates to a process of α,β-desaturation of compounds containing a carbonyl moiety, in particular, ketones, esters, amides, lactones, lactams and aldehydes. The process comprising the following steps in the following order: a) preparing a stable enol derivative from the compound comprising a carbonyl moiety; b) preparing a mixture comprising the enol derivative obtained in step a), a base, an electrolyte and a solvent; c) electrolyzing the mixture obtained in step b).

## Description

The present invention relates to a new process of α,β-desaturation of compounds containing a carbonyl moiety, in particular, ketones, esters, amides, lactones, lactams and aldehydes.

### BACKGROUND OF THE INVENTION

α,β-Unsaturated carbonyl compounds are versatile intermediates in the synthesis of pharmaceuticals and biologically active compounds.

α,β-Unsaturated carbonyl compounds may be synthetized from compounds comprising a carbonyl moiety according to the following scheme:

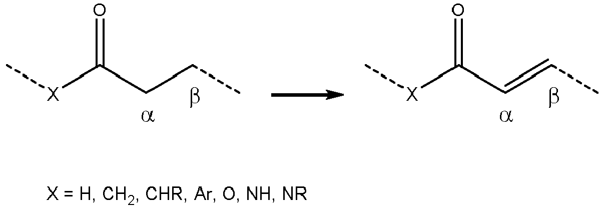

Many synthetic methods to access such structures have been disclosed, including method using hypervalent iodine reagents or method using transition metals reagents and catalysts (S.S. Stahl & T. Diao, Comprehensive Organic Synthesis II, 2014, Vol. 7, p. 178-212). Electrochemical methods have been also used but with a limited scope and low yields (T. Shono et al., J. Am. Chem. Soc. 1974, 96, 3532-3536; T. Shono et al., J. Am. Chem. Soc. 1975, 97, 6144-6147; J. B. Sperry et al., J. Org. Chem. 2004, 69, 3726-3734).

More recently other methods have been disclosed, such as, for example, palladium-catalyzed α,β-dehydrogenation of esters, nitriles (Y. Chen et al., J. Am. Chem. Soc. 2015, 137, 5875-5878) amides (Y. Chen et al., J. Am. Chem. Soc. 2016, 138, 1166-1169) and cyclic cetones (D. Huang et al., Org. Lett. 2018, 20, 684-687) via their zinc enolates, platinum-catalyzed desaturation of lactams, ketones and lactones (M. Chen et al., Angew. Chem. Int. Ed., 2018, 57, 16205-16209), radical α,β-dehydrogenation of saturated amides via α-oxidation with TEMPO under transition metal-free conditions (M.-M. Wang et al., J. Org. Chem., 2019, 84, 8267-8274) or copper-catalyzed desaturation of lactones, lactams, and ketones under pH-neutral conditions (M. Chen et al., J. Am. Chem. Soc., 2019, 141, 14889-14897).

However, these methods are not practical for industrial processes. Indeed, these methods provide require unsafe reagents or a large amount of expensive catalysts and/or expensive ligands.

Therefore, there is still a need for a process of α,β-desaturation of compounds containing a carbonyl moiety, in particular, ketones, esters, amides, lactones, lactams and aldehydes that is inexpensive, while being inherently safe and scalable.

### SUMMARY OF THE INVENTION

The inventors have now succeeded in developing a novel process of α,β-desaturation of a compound comprising a carbonyl moiety that is viable at an industrially relevant scale.

The present invention therefore relates to a process of α,β-desaturation of a compound comprising a carbonyl moiety comprising the following steps in the following order:
a) preparing a stable enol derivative from the compound comprising a carbonyl moiety;
b) preparing a mixture comprising the enol derivative obtained in step a), a base, an electrolyte and a solvent;
c) electrolyzing the mixture obtained in step b).

### DETAILED DESCRIPTION OF THE INVENTION

The present invention relates to a process of α,β-desaturation of a compound comprising a carbonyl moiety comprising the following steps in the following order:
a) preparing a stable enol derivative from the compound comprising a carbonyl moiety;
b) preparing a mixture comprising the enol derivative obtained in step a), a base, an electrolyte and a solvent;
c) electrolyzing the mixture obtained in step b).

The term "α,β-desaturation" as used herein must be understood as referring to the introduction of a double bond C=C in a compound comprising a carbonyl moiety between the two carbon atoms located in positions α and β with respect to the carbonyl moiety.

The term "compound comprising a carbonyl moiety" as used herein must be understood as referring to any compound comprising a C=O group. In particular, the compound comprising a carbonyl moiety is selected from ketones, esters, amides, lactones, lactams and aldehydes. More particularly, the compound comprising a carbonyl moiety is selected from ketones, esters, lactones, lactams and aldehydes. Even more particularly, the compound comprising a carbonyl moiety is a ketone.

In other words, the compound comprising a carbonyl moiety may be defined by the formula I: 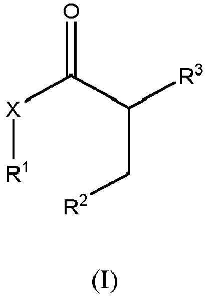 wherein
X is selected from H, CH₂, CHR⁴, aryl, O, NH and NR⁵; preferably X is selected from CH₂, CHR⁴, aryl and O;
**R¹** is inexistent when X is H or **R¹** is selected from H and C1-C6-alkyl; preferably **R¹** is C1-C6-alkyl; more preferably **R¹** is C1-C4-alkyl; still more preferably **R¹** is C1-C2-alkyl;
**R²** is selected from H, C1-C6-alkyl and C3-C6-cycloalkyl; preferably **R²** is selected from H, C1-C4-alkyl and C3-C6-cycloalkyl; more preferably **R²** is H or C3-C6-cycloalkyl; still more preferably **R²** is H or C3-cycloalkyl; or
**R¹** and **R²** form together a 4- to 15-membered cycle; preferably **R¹** and **R²** form together a 5- to 15-membered cycle;
**R³** is selected from H, C1-C6-alkyl and aryl; preferably **R³** is selected from H, C1-C4-alkyl and aryl; more preferably **R³** is H or aryl; still more preferably **R³** is H or phenyl; or
**R²** and **R³** form together a 6-membered cycle or heterocycle optionally substituted by C1-C6-alkyloxycarbonyl;
**R⁴** is C1-C6-alkyl or aryl; preferably **R⁴** is aryl; more preferably **R⁴** is phenyl; and
**R⁵** is C1-C6-alkyl or C1-C6-alkyloxycarbonyl; preferably **R⁵** is C1-C6-alkyloxycarbonyl; more preferably **R⁵** is C1-C4-alkyloxycarbonyl.

In one embodiment, the compound comprising a carbonyl moiety is none of the following compounds:
(13S)-3-hydroxy-13-methyl-6,7,8,9,11,12,13,14,15,16-decahydro-17H-cyclopenta[a]phenanthren-17-one, and
(13S)-3-((tert-butyldimethylsilyl)oxy)-13-methyl-6,7,8,9,11,12,13,14,15,16-decahydro-17H-cyclopenta[a]phenanthren-17-one.

The step a) of the process of the invention consists in preparing an enol derivative from the compound comprising a carbonyl moiety.

In particular, the enol derivative of step a) of the process of the invention is selected from silyl ether enols, enol phosphates and enol esters. More particularly, the enol derivative of step a) of the process of the invention is selected from silyl ether enols, enol phosphates. Still more particularly, the enol of step a) of the process of the invention is a silyl ether enol.

In other words, the enol derivative of step a) of the process of the invention may be defined by the formula II: 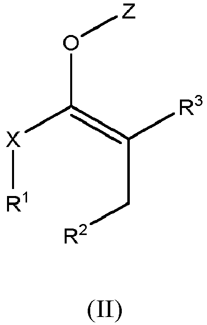 wherein
**X** is selected from H, CH₂, CHR⁴, aryl, O, NH and NR⁵; preferably **X** is selected from CH₂, CHR⁴, aryl and O;
**R¹** is inexistent when **X** is H or **R¹** is selected from H and C1-C6-alkyl; preferably **R¹** is C1-C6-alkyl; more preferably **R¹** is C1-C4-alkyl; still more preferably **R¹** is C1-C2-alkyl;
**R²** is selected from H, C1-C6-alkyl and C3-C6-cycloalkyl; preferably **R²** is selected from H, C1-C4-alkyl and C3-C6-cycloalkyl; more preferably **R²** is H or C3-C6-cycloalkyl; still more preferably **R²** is H or C3-cycloalkyl; or
**R¹** and **R²** form together a 4- to 15-membered cycle; preferably **R¹** and **R²** form together a 5- to 15-membered cycle;
**R³** is selected from H, C1-C6-alkyl and aryl; preferably **R³** is selected from H, C1-C4-alkyl and aryl; more preferably **R³** is H or aryl; still more preferably **R³** is H or phenyl; or
**R²** and **R³** form together a 6-membered cycle or heterocycle optionally substituted by C1-C6-alkyloxycarbonyl;
**R⁴** is C1-C6-alkyl or aryl; preferably **R⁴** is aryl; more preferably **R⁴** is phenyl;
**R⁵** is C1-C6-alkyl or C1-C6-alkyloxycarbonyl; preferably **R⁵** is C1-C6-alkyloxycarbonyl; more preferably **R⁵** is C1-C4-alkyloxycarbonyl; and
**Z** is selected from Si(C1-C4-alkyl)₃, P(O)(O-C1-C4-alkyl)₂ and P(O)(OAr)₂; preferably **Z** is selected from Si(C1-C4-alkyl)₃, P(O)(O-C1-C4-alkyl)₂ and P(O)(OPh)₂; more preferably **Z** is selected from Si(C1-C4-alkyl)₃ and P(O)(OPh)₂; still more preferably **Z** is selected from Si(C1-C2-alkyl)₃ and P(O)(OPh)₂.

The enol derivative may be prepared by different ways with reactions known by the person skilled in the art.

In particular, the enol derivative may be prepared by reacting the compound comprising a carbonyl moiety with a silyl reagent or a phosphate reagent in an organic solvent in the presence of a base.

Advantageously, the silyl reagent may be selected from tri-C1-C4-alkylsilyl halides and tri-C1-C4-alkylsilyl trihalo-C1-C4-alkylsulfonates. Particularly the silyl reagent is selected from tri-C1-C4-alkylsilyl halides. More particularly, the silyl reagent may be trimethylsilyl chloride (TMSC1).

Advantageously, the phosphate reagent may be selected from di-C1-C4-alkyl phosphoryl halides and diaryl phosphoryl halides. Particularly, the phosphate reagent may be selected from di-C1-C4-alkyl phosphoryl chloride and diaryl phosphoryl chloride. More particularly, the phosphate reagent may be diphenyl phosphoryl chloride

In particular, the solvent used in step a) of the process of the invention may be a polar aprotic solvent. Particularly, the solvent used in step a) of the process of the invention may be selected from acetonitrile, dichloromethane, *N*,*N-*dimethylacetamide, *N,N-*dimethylformamide, *N*-methylpyrrolidone, tetrahydrofuran, 2-methyl-tetrahydrofuran and mixtures thereof. Still more particularly, the solvent used in step a) of the process of the invention may be tetrahydrofuran.

In particular, the base used in step a) of the process of the invention may be any base known by the skilled person in the art. More particularly, the base used in step a) may be a strong base. Still more particularly, the base used in step a) may be selected from lithium bis(trimethylsilyl)amide (LiHMDS), sodium bis(trimethylsilyl)amide (NaHMDS), potassium bis(trimethylsilyl)amide (KHMDS), lithium diisopropylamide (LDA), n-butyllithium, triethylamine (Et₃N), di-isopropylethylamine (DIPEA), pyridine, collidine, lutidine, sodium hydride, sodium hydroxide, potassium hydroxide and lithium hydroxide.

Even more particularly, the base used in step a) may be lithium bis(trimethylsilyl)amide (LiHMDS).

In particular, the step a) of the process of the invention may be performed at a temperature ranging from -150°C to 0°C, preferably from -100°C to -50°C, more preferably from - 90°C to -60°C, still more preferably the step a) of the process of the invention may be performed at a temperature of about -78°C.

After the reaction of the step a) of the process of the invention is ended, the resulting product may be purified using the procedures known by the person skilled in the art, such as, for example, recrystallization or purification by column chromatography, preparative thin layer chromatography or distillation.

Once formed, the enol derivative is then used in the step b) of the process of the invention.

The **step b)** of the process of the invention consists in preparing a mixture comprising the enol derivative obtained in step a), a base, an electrolyte and a solvent.

In particular, the base used in step b) of the process of the invention may be selected from 2,6-lutidine, 1,8-diazabicyclo[5.4.0]undec-7-ene (DBU) and 2,4,6-collidine. More particularly, the base used in step b) of the process of the invention is 2,4,6-collidine.

Advantageously, the amount of base used in step b) is from 10% v/v to 50% v/v with respect to the total volume of the mixture. Preferably, the amount of base used in step b) is from 15% v/v to 45% v/v with respect to the total volume of the mixture. More preferably, the amount of base used in step b) is from 20% v/v to 40% v/v with respect to the total volume of the mixture. Still more preferably, the amount of base used in step b) is from 25% v/v to 35% v/v with respect to the total volume of the mixture. Even more preferably, the amount of base used in step b) is about 30% v/v with respect to the total volume of the mixture.

In particular, the electrolyte used in step b) of the process of the invention may be selected from NaSbF₆, LiClO₄, LiBF₄ and NaOTs. More particularly, the electrolyte used in step b) of the process of the invention is NaSbF₆.

Advantageously, the concentration of electrolyte used in step b) is from 0.01 M to 0.30 M, preferably from 0.05 M to 0.25 M, more preferably from 0.10 M to 0.20 M, in the mixture. Still more preferably, the concentration of electrolyte used in step b) is about 0.15 M in the mixture.

In particular, the solvent used in step b) of the process of the invention may be a polar aprotic solvent. More particularly, the solvent used in step b) of the process of the invention may be selected from acetonitrile, dichloromethane, acetone, ethyl acetate, *N,N-*dimethylacetamide, *N*,*N*-dimethylformamide, *N*-methylpyrrolidone, tetrahydrofuran, 2-methyl-tetrahydrofuran and mixtures thereof. Still more particularly, the solvent used in step b) of the process of the invention is acetonitrile.

Once prepared, the mixture is used in step c) of the process of the invention.

The **step c)** of the process of the invention consists in electrolyzing the mixture obtained in step b).

The step c) of the process of the invention allows the α,β-desaturation of a compound comprising a carbonyl moiety and a α,β-unsaturated carbonyl compound is obtained, as illustrated by the following scheme:

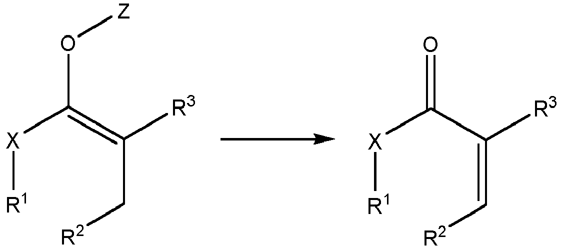

The electrolysis may be performed by any technique known by the person skilled in the art.

Typically, the mixture obtained in step b) of the process of the invention is contacted with an anode and a cathode and a constant current is applied.

The anode and the cathode may be of any kind known by the person skilled in the art. In particular, the anode and the cathode may be made of carbon graphite.

In particular, the step c) may be performed under a constant current ranging from 1 to 20 mA, more particularly from 5 to 15 mA. Still more particularly, the step c) may be performed under a constant current of about 10 mA.

Advantageously, the step b) of the process of the invention is performed at a temperature ranging from 10°C to 50°C, preferably the step c) of the process of the invention is performed at room temperature, i.e. at a temperature of about 18°C to 25°C.

At the end of the reaction occurring during step c), the reaction mixture may be quenched using the procedures known by the person skilled in the art, such as, for example, adding an aqueous solution of hydrochloric acid, optionally followed by extraction with an organic solvent.

After the reaction of the step c) of the process of the invention is ended, the resulting product may be purified using the procedures known by the person skilled in the art, such as, for example, recrystallization or purification by column chromatography, preparative thin layer chromatography or distillation.

In one embodiment, steps a) and b) of the process of the invention are performed separately. In this case, the enol derivative obtained at the end of step a) is isolated and then used in step b).

In another embodiment, steps a), b) and c) of the process of the invention are performed successively in one pot. In this case the enol derivative obtained at the end of step a) is not isolated and directly used in step b).

In another embodiment, steps b) and c) of the process of the invention are performed in flow using the procedures known by the person skilled in the art.

### DEFINITIONS

The definitions and explanations below are for the terms as used throughout the entire application, including both the specification and the claims.

Unless otherwise stated any reference to compounds of the invention herein, means the compounds as such as well as their pharmaceutically acceptable salts and solvates.

When describing the process and compounds of the invention, the terms used are to be construed in accordance with the following definitions, unless indicated otherwise.

The term "alkyl" by itself or as part of another substituent refers to a hydrocarbyl radical of Formula CₙH₂ₙ₊₁ wherein n is an integer greater than or equal to 1.

The term "alkenyl" by itself or as part of another substituent refers to a hydrocarbyl radical formed from an alkene by removal of one hydrogen atom of Formula CₙH₂ₙ₋₁ wherein n is an integer greater than or equal to 2.

The term "halo" or "halogen" means fluoro, chloro, bromo, or iodo.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoromethyl, 1,1,1-trifluoroethyl and the like. Preferred haloalkyl group is trifluoromethyl.

The term "aryl" as used herein refers to a polyunsaturated, aromatic hydrocarbyl group having a single ring (*i.e.* phenyl) or multiple aromatic rings fused together (e.g. naphthyl), typically containing 5 to 12 atoms; preferably 6 to 10, wherein at least one ring is aromatic. Examples of aryl groups include but are not limited to phenyl, naphthyl, anthracyl. Preferred aryl group according to the invention is phenyl.

The term "alkoxy" as used herein refers to a group -O-alkyl, wherein alkyl is as herein defined. Examples of alkoxy groups include but are not limited to methoxy, ethoxy, n-propyloxy, isopropyloxy, *n*-butyloxy, *t*-butyloxy, *sec*-butyloxy and *n*-pentyloxy.

The term "carbonyl" or "carbonyl group" as used herein refers to a functional group composed of a carbon atom double-bonded to an oxygen atom: C=O, also defined by C(O).

The term "sulfonyl" as used herein refers to a functional group composed of a sulfur atom double-bonded to two oxygen atoms: O=S=O, also defined by SO₂.

The term "alkyloxycarbonyl" as used herein refers to a group -C(O)-alkoxy, wherein alkoxy is as herein defined. Non-limiting examples of alkyloxycarbonyl include *tert-*butyloxycarbonyl (abbreviation: Boc).

The term "haloalkylsulfonyl" as used herein refers to a group -SO₂-haloalkyl, wherein haloalkyl is as herein defined. Non-limiting examples of haloalkylsulfonyl include trifluoromethylsulfonyl (abbreviation: Tf).

The term "polar aprotic solvents" as used herein refers to solvents having large dipole moments, i.e. molecules having bonds between atoms with very different electronegativities, while being unable to participate in hydrogen bonding. Non-limiting examples of polar aprotic solvents include dichloromethane, *N*-methylpyrrolidone, tetrahydrofuran, 2-methyl-tetrahydrofuran, ethyl acetate, acetone, *N*,*N-*dimethylformamide, acetonitrile, dimethylsulfoxide, *N*,*N*-dimethylacetamide, 1,3-dimethyl-2-imidazolidinone and *N*,*N'*-dimethylpropyleneurea.

The term "non-polar solvents" as used herein refers to solvents having dipole moment equal or close to zero, i.e. molecules having no polar group or molecules comprising polar groups but whose geometry causes the dipole moment to vanish. Non-limiting examples of non-polar solvents include hexane, cyclohexane, benzene, toluene, xylene, 1,4-dioxane, chloroform and diethyl ether. Non-limiting examples of aromatic non-polar solvents include benzene, toluene and xylene.

The present invention will be better understood with reference to the following examples and figures. These examples are intended to be representative of specific embodiments of the invention and are not intended as limiting the scope of the invention.

### EXAMPLES

### General experimental

Tetrahydrofuran (THF), dichloromethane (CH₂Cl₂), *N*,*N-*dimethylformamide (DMF), and acetonitrile (MeCN) were obtained by passing the previously degassed solvents through an activated alumina column. NaSbF₆ (technical grade) was purchased from Sigma-Aldrich and was carefully grinded prior to use. 2,4,6-collidine (99%) was purchased from Sigma-Aldrich and Alfa Aesar. All the other reagents were purchased at the highest commercial quality and used without further purification, unless otherwise stated.

Yields refer to chromatographically and spectroscopically (GC-MS) homogeneous material.

TLC was performed using 0.25 mm E. Merck Silica plates (60F-254), using short-wave UV light for visualization, and phosphomolybdic acid, Ce(SO₄)₂, acidic ethanolic anisaldehyde, or KMnO₄ as developing agents upon heating.

¹H-NMR and ¹³C-NMR spectra were recorded on Bruker DRX-600, DRX-500, and AMX-400 instruments and are calibrated using residual undeuterated solvent (CHCl₃ at 7.26 ppm ¹H-NMR, 77.16 ppm ¹³C-NMR). The following abbreviations were used to explain multiplicities: s = singlet, d = doublet, t = triplet, q = quartet, m = multiplet, br = broad. Column chromatography was performed using E. Merck silica gel (60, particle size 0.043-0.063 mm).

High-resolution mass spectra (HRMS) were recorded on Waters LC with G2-XS TOF mass spectrometer by electrospray ionization time of flight reflectron experiments.

GC-MS (EI) was recorded on Agilent 7820A GC systems and 5975 Series MSD.

All temperatures are expressed in °C and all reactions were carried out at room temperature (RT) unless otherwise stated.

The following abbreviations are used:
- GC-MS:: gas chromatography-mass spectrometry
- HRMS:: high-resolution mass spectrometry
- LiHMDS:: lithium bis(trimethylsilyl)amide
- MeCN:: acetonitrile
- MTBE:: methyl *tert*-butyl ether
- NMR:: Nuclear Magnetic Resonance
- RT:: room temperature
- THF:: tetrahydrofuran
- TLC:: thin layer chromatography
- TMSCl:: trimethylsilyl chloride
- TMSOTf:: trimethylsilyl trifluoromethanesulfonate

### General procedure A1 for the formation of silyl enol ether

Compound comprising a carbonyl moiety (1.0 equiv) was dissolved in dry THF under N₂ flow. The resulting solution was cooled to -78°C and LiHMDS (1M in THF, 1.2 equiv) was added over 30 min at -78°C. The resulting brown slurry was quenched by addition of TMSCl or diphenyl phosphoryl chloride (1.3 equiv) at -78°C. The reaction mixture was then warmed to RT and stirred for 1 h (reaction monitored by TLC). The solvent was removed under reduced pressure and the resulting crude product was then purified by flash chromatography on SiO₂ to furnish the desired silyl enol ether.

### General procedure A2 for the formation of silyl enol ether

NaI (1.2 equiv) was placed in a round bottom flask, flame dried under high vacuum, and allowed to cool to ambient temperature under argon. MeCN was added to dissolve the NaI. To the solution was added cyclopentadecanone (1.00 equiv), followed by Et₃N (1.55 equiv). The flask was fitted with an addition funnel, and the funnel was charged with TMSCl (1.3 equiv), which was added dropwise over 30 min. The resulting suspension was stirred for an additional 2 hours at ambient temperature. Hexane was added, and the biphasic system was stirred vigorously for 10 min. The phases were separated and the MeCN layer was extracted with hexane (three times). The combined hexane phases were washed with saturated NaHCO₃ and brine, dried over Na₂SO₄, filtered, and concentrated under reduced pressure to afford the desired product.

### Enol 1

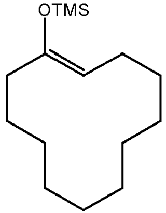

Following the general procedure A2 on 920 mg (5.05 mmol) scale of the corresponding ketone at RT. The resulting crude product was purified by column chromatography on silica gel (99:1 Hex:EtOAc) to afford Enol 1 in 73% yield (937 mg, 3.67 mmol) as a colorless oil. ¹H NMR (600 MHz, CDCl₃): δ 4.56 (t, J = 7.3 Hz, 1H), 2.07 - 2.01 (m, 4H), 1.58 - 1.42 (m, 4H), 1.37 - 1.29 (m, 12H), 0.19-0.21 (s, 9H). The NMR data were consistent with those previously reported (J. J. Song et al., Org. Lett. 2008, 10, 877-880).

### Enol 2

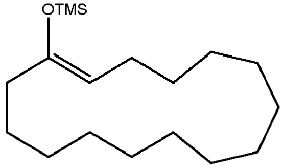

Following the general procedure A2 on 1 g (4.50 mmol) scale of the corresponding ketone at 40°C. The resulting crude product was purified by column chromatography on silica gel (Hexane) to afford Enol 2 in 98% yield (1.36 g, 4.41 mmol) as a colorless oil. ¹H NMR (500 MHz, CDCl₃): δ 4.63 (t, J = 7.6 Hz, 1H), 2.09 - 2.05 (m, 2H), 1.96 (q, J = 7.3 Hz, 2H), 1.38 - 1.34 (m, 22H), 0.20 (s, 9H).

### Enol 3

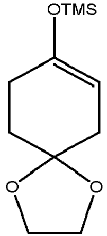

Following the general procedure A2 on 1.1 g (7.05 mmol) scale of the corresponding ketone at 40°C. The resulting crude product was purified by column chromatography on silica gel (95:5 Hexane:EtOAc) to afford Enol 3 in 84% yield (1.37 g, 5.82 mmol) as a colorless oil. ¹H NMR (400 MHz, CDCl₃): δ 4.72 (t, J = 3.8 Hz, 1H), 3.98 - 3.95 (m, 5H), 2.27 - 2.19 (m, 2H), 1.80 (t, J = 6.6 Hz, 2H), 0.18 (s, 9H). The NMR data were consistent with those previously reported (W. J. Kerr et al., Org. Lett. 2001, 3, 2945-2948).

### Enol 4

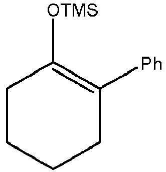

Following the literature procedure (C. H. Cheon et al., J. Am. Chem. Soc. 2011, 133, 13248-13251) on 0.87 g (5 mmol) scale of the corresponding ketone. The resulting crude product was purified by column chromatography on silica gel (95:5 Hexane:EtOAc) to afford Enol 4 in 93% yield (1.14 g, 4.65 mmol) as a colorless oil. ¹H NMR (400 MHz, CDCl₃): δ: 7.34 - 7.36 (dd, J = 1.3, 8.2 Hz, 2H), 7.25 - 7.28 (m, 2H), 7.12 - 7.15 (m, 1H), 2.34 - 2.38 (m, 2H), 2.15 - 2.19 (m, 2H), 1.64 - 1.77 (m, 4H), -0.05 (s, 9H). The NMR data were consistent with those previously reported.

### Enol 5

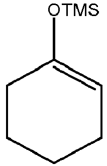

Following the literature procedure (L. R. Mills et al., Org. Lett. 2019, 21, 8805-8809) on 196 mg (2 mmol) scale of the corresponding ketone. The resulting crude product was purified by column chromatography on silica gel (98:2 Hexane:EtOAc) to afford Enol 5 in 81% yield (275 mg, 1.62 mmol) as a colorless oil. ¹H NMR (400 MHz, CDCl₃): 0.18 (s, 9H), 1.48-1.54 (m, 2H), 1.63-1.69 (m, 2H), 1.97-2.03 (m, 4H), 4.86-4.88 (m, 1H). The NMR data were consistent with those previously reported.

### Enol 6

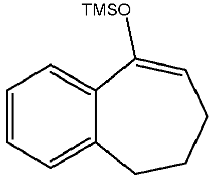

Following the general procedure A2 on 820 mg (5.12 mmol) scale of the corresponding ketone at 40°C. The resulting crude product was purified by column chromatography on silica gel (95:5 Hexane:EtOAc) to afford Enol 6 in 76% yield (903 g, 3.89 mmol) as a colorless oil. ¹H NMR (500 MHz, CDCl₃): δ 7.51 (dd, J = 7.5, 1.5 Hz, 1H), 7.28 - 7.17 (m, 3H), 5.45 (t, J = 6.7 Hz, 1H), 2.68 (t, J = 6.6 Hz, 2H), 2.05 (td, J = 7.1, 5.9 Hz, 2H), 1.97 - 1.92 (m, 2H), 0.21 (s, 9H).

### Enol 7

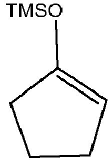

Following the literature procedure (A. Y. Hong et al., Angew. Chem. Int. Ed. 2011, 50, 2756-2760) on 252 mg (3 mmol) scale of the corresponding ketone. The resulting crude product was purified by column chromatography on silica gel (Hexane) to afford Enol 7 in 95% yield (444 mg, 2.85 mmol) as a colorless oil. ¹H NMR (400 MHz, CDCl₃): 0.20 (s, 9H), 1.82-1.90 (m, 2H), 2.24-2.29 (m, 4H), 4.62-4.63 (m, 1H).

### Enol 8

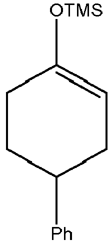

Following the general procedure A2 on 1.7 g (10 mmol) scale of the corresponding ketone. The resulting crude product was purified by column chromatography on silica gel (98:2 Hexane:EtOAc) to afford Enol 8 in 72% yield (1.77 g, 7.2 mmol) as a colorless oil. ¹H NMR (600 MHz, CDCl₃): δ 7.34 (dd, J = 8.0, 7.1 Hz, 2H), 7.29 - 7.28 (m, 1H), 7.27 - 7.24 (m, 1H), 4.99 (dt, J = 4.7, 1.9 Hz, 1H), 2.80 (dddd, J = 14.9, 10.0, 5.1, 2.9 Hz, 1H), 2.34 - 2.12 (m, 3H), 2.01 - 1.92 (m, 1H), 1.90 (dddd, J = 12.8, 11.7, 10.8, 5.7 Hz, 1H), 0.26 (s, 9H). The NMR data were consistent with those previously reported (J. Francos et al., Dalton Trans. 2014, 43, 1408-1412).

### Enol 9

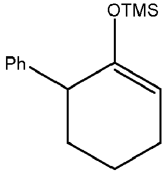

Following the literature procedure (S. L. Poe et al., Angew. Chem. Int. Ed. 2011, 50, 4189-4192) on 174 mg (1 mmol) scale of the corresponding ketone. The resulting crude product was purified by column chromatography on silica gel (98:2 Hexane:EtOAc) to afford Enol 9 in 89% yield (219 gm, 0.89 mmol) as a colorless oil. ¹H NMR (500 MHz, CDCl₃): δ 0.02 (9H, s); 1.42-1.50 (1H, m), 1.52-1.60 (1H, m), 1.66-1.74 (1H, m), 1.96-2.04 (1H, m), 2.05-2.18 (2H, m), 3.34 (1H, t, J = 5.6 Hz), 5.05 (1H, t, J = 3.9), 7.14-7.22 (3H, m), 7.24-7.28 (2H, m).

### Enol 10

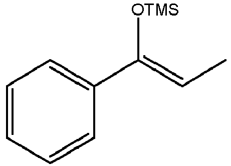

Following the literature procedure (N. Singhal et al., J. Am. Chem. Soc. 2005, 127, 14375-14382) on 268 mg (2 mmol) scale of the corresponding ketone. The resulting crude product was purified by column chromatography on silica gel (95:5 Hexane:EtOAc) to afford Enol 10 in 83% yield (342 mg, 1.66 mmol) as a colorless oil. ¹H NMR (400 MHz, CDCl₃): δ 7.49 - 7.42 (m, 2H), 7.33 - 7.19 (m, 3H), 5.33 (q, J = 6.9 Hz, 1H), 1.74 (d, J = 6.9 Hz, 3H), 0.14 (s, 9H).

### Enol 11

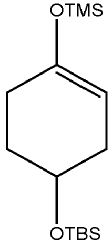

Following the literature procedure (W. J. Kerr et al., Synlett 2008, 9, 1386-1390) on 1.14 g (5 mmol) scale of the corresponding ketone at 40°C. The resulting crude product was purified by column chromatography on silica gel (99:1 Hexane:EtOAc) to afford Enol 11 in 80% yield (1.2 g, 4 mmol) as a colorless oil. ¹H NMR (400 MHz, CDCl₃): δ 0.06 (s, 3H), 0.07 (s, 3H), 0.18 (s, 9H), 0.89 (s, 9H), 1.60-1.83 (m, 2H), 1.97-2.16 (m, 3H), 2.17-1.27 (m, 1H), 3.84-3.93 (m, 1H), 4.66-4.73 (m, 1H).

### Enol 12

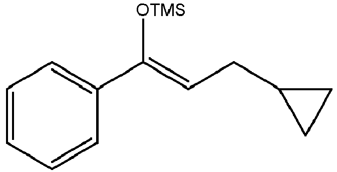

Following the general procedure A2 on 250 mg (1.43 mmol) scale of the corresponding ketone at RT. The resulting crude product was purified by column chromatography on silica gel (98:2 Hexane:EtOAc) to afford Enol 12 in 81% yield (286 mg, 1.16 mmol) as a colorless oil. ¹H NMR (600 MHz, CDCl₃): δ 7.52 - 7.48 (m, 1H), 7.34 - 7.24 (m, 2H), 5.37 (m, 1H), 2.16 (t, J = 6.9 Hz), 1.77 (d, J = 6.9 Hz) (2H), 0.83 - 0.81 (m, 1H), 0.49 - 0.46 (m, 2H), 0.16 (m, 11H).

### Enol 13

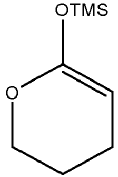

Following the literature procedure (L. Pedzisa et al., Tetrahedron Lett. 2008, 49, 4142-4144) on 200 mg (2 mmol) scale of the corresponding lactone. The resulting crude product was purified by column chromatography on basic alumina (70:30 Hexane:EtOAc) to afford Enol 13 in 55% yield (411 mg, 1.1 mmol) as a colorless oil. ¹H NMR (600 MHz, CDCl₃): δ 7.00 - 6.93 (m, 1H), 6.07 (dt, J = 9.8, 1.9 Hz, 1H), 4.45 (t, J = 6.3 Hz, 1H), 2.48 (tdd, J = 6.2, 4.2, 1.8 Hz, 2H).

### Enol 14

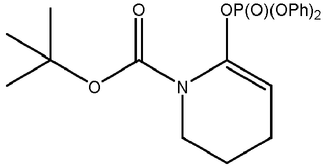

Following general procedure A1 on 400 mg (1.96 mmol) scale of the corresponding lactam. The resulting crude product was purified by column chromatography on basic alumina basic alumina (70:30 Hexane:EtOAc with 1% Et₃N) to afford Enol 14 in 78% yield (665 mg, 1.52 mmol) as a colorless oil. Note: Enol 14 is stable for a couple of hours and should be used immediately. ¹H NMR (600 MHz, CDCl₃): δ 7.41 - 7.33 (m, 4H), 7.31 - 7.24 (m, 4H), 7.23 - 7.16 (m, 2H), 5.12 (td, J = 3.8, 2.9 Hz, 1H), 3.82 - 3.51 (m, 2H), 2.19 (tt, J = 6.8, 4.1 Hz, 2H), 1.88 - 1.64 (m, 4H), 1.45 (m, 9H).

### Enol 15

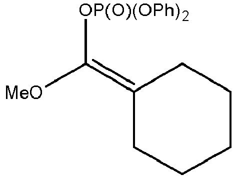

Following general procedure A1 on 1.0 g (7.01 mmol) scale of the corresponding ester. The resulting crude product was purified by column chromatography on basic alumina basic alumina (85:15 Hexane:EtOAc with 1% Et₃N) to afford Enol 15 in 81% yield (1.9 g, 5.37 mmol) as a colorless oil. ¹H NMR (400 MHz, CDCl₃): δ 7.38 - 7.34 (m, 4H), 7.29 (dt, J = 7.6, 1.3 Hz, 4H), 7.22 (ddt, J = 7.4, 5.6, 1.1 Hz, 2H), 3.64 (s, 3H), 2.17 - 2.14 (m, 2H), 2.05 (dt, J = 7.1, 2.4 Hz, 2H), 1.52 - 1.45 (m, 6H).

### Enol 16

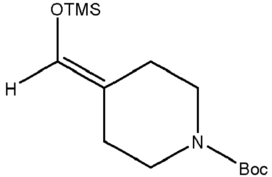

Following the general procedure A2 on 2.13 g (10 mmol) scale of the corresponding ketone at 40 °C. The resulting crude product was purified by column chromatography on silica gel (90:10 Hexane:EtOAc) to afford Enol 16 in 14% yield (390 mg, 0.14 mmol) as a white solid. ¹H NMR (400 MHz, CDCl₃): δ 6.10 (t, J = 1.3 Hz, 1H), 3.39-3.33 (m, 4H), 2.25 (t, J = 5.8 Hz, 2H), 2.01 (t, J = 5.8 Hz, 2H), 1.46 (s, 9H), 0.17 (s, 9H).

### General procedure B for electrochemical a,β-desaturation of ketones, esters, lactams and aldehyde: General procedure for small scale using IKA ElectraSyn 2.0:

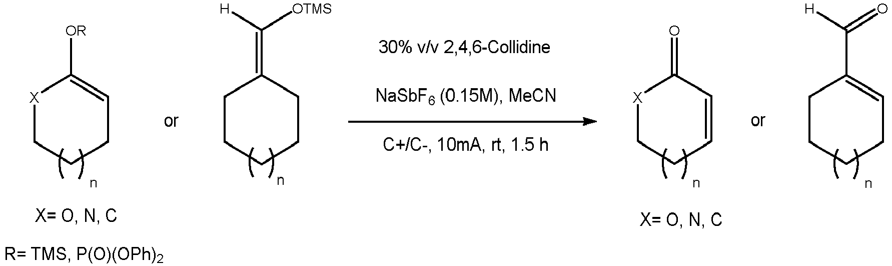

Unless otherwise specified, the reaction carried out on 0.2 mmol scale. The Electra-Syn vial (5 mL) was equipped with a stir bar and charged with the corresponding enol (0.2 mmol) obtained according to general procedure A. MeCN (2.1 mL) and 2,4,6-collidine (900 µL) were added to the vial following by the addition of solid NaSbF₆ (116 mg, 0.15M). The Electra-Syn vial cap equipped with anode (graphite) and cathode (graphite) was inserted into the reaction mixture. After pre-stirring for 10 minutes under argon, the reaction mixture was electrolyzed under a constant current of 10 mA for 1.5 hours. The reaction was monitored by GC-MS or TLC. After completion, the ElectraSyn vial cap was removed, and the electrodes were rinsed with EtOAc (10 mL). Then, the reaction mixture was diluted with EtOAc (100 mL) and extracted with 1M HCl (100 mL) and brine (100 mL). The organic layer was dried over Na₂SO₄ and evaporated under reduced pressure. The crude residue was purified by silica gel column chromatography.

### General procedure C for electrochemical a,β-desaturation of ketones: General procedure for one-pot reaction using IKA ElectraSyn 2.0:

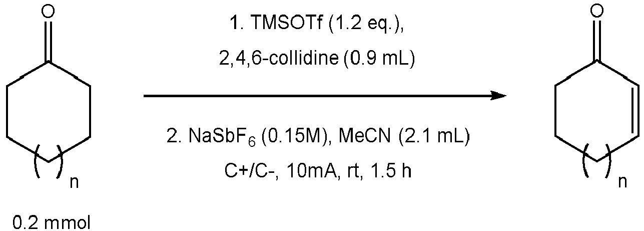

Under an argon atmosphere, to a flame-dried electrochemical cell without electrodes charged with ketone (0.20 mmol) and dry 2,4,6-collidine (dried over 3Å molecular sieves, 0.9 mL) was added TMSOTf (1.2 equiv, 0.24 mmol), and resulting solution was stirred at room temperature. After consumption of starting material as judged by TLC (typically < 5 min), MeCN (2.1 mL) and NaSbF₆ (0.15 M, 0.45 mmol) was added to the reaction mixture. The vial cap equipped with a pair of graphite electrodes was screwed on, and headspace of the vial was purged with argon. After pre-stirring for 5 min, the mixture was electrolyzed with a set of parameters as follows: constant current, 10 mA; no reference electrode; time, 1.5 h; 0.20 mmol substrate, 2.8 F/mol; no alternate polarity; stirring, 1,500 rpm.

The reaction mixture was quenched by 1.0 M HCl and organic materials were extracted with EtOAc, and then combined organic layers were washed with brine, dried over anhydrous Na₂SO₄, and concentrated under reduced pressure. Preparative thin-layer chromatography gave the corresponding enone.

### General procedure D for electrochemical a,β-desaturation of ketones, esters, lactams and aldehyde: General procedure for gram scale:

To a clean 250 mL beaker was added NaSbF₆ (30 mmol, 0.15 M) and 140 mL of MeCN. Stirring continued until a clear solution was obtained. Then TMS-enol ether (13.5 mmol, 1.0 equiv.) and 60 mL of 2,4,6-collidine were added into the beaker and the reaction mixture was allowed to stir at room temperature until homogeneous. Then the solution was allowed to undergo electrolysis with one graphite electrode as anode and two graphite electrodes as cathodes. The area of each electrode immersed in the reaction mixture was 30 cm². The reaction progress was monitored with TLC as well as GC.

Upon completion of the reaction, the reaction mixture was transferred to a 1L conical flask and 300 mL of 3M HCl was added dropwise. The mixture was extracted with MTBE (3×300 mL) and the combined organic phases were dried and concentrated to give the crude product. The crude product was further purified via column chromatography.

### General procedure E for electrochemical a,β-desaturation of ketones, esters, lactams and aldehyde: General procedure for 100 gr in flow:

**Frame Cell Setup** (Two sets of same, independent 3-in-one frame cells combined): Six Teflon frame blocks (length: 30.0 cm, width: 20.0 cm, thickness: 2.0 cm) were packed in a row. Eight graphite plates (length: 34.0 cm, width: 20.0 cm, thickness: 2.0 mm) as cathodes and anodes which have two silica pads (length: 30.0 cm, width: 20.0 cm, thickness: 2.0 mm) attached on their both sides were inserted between each frame. The two ends of frame cell were attached by two Teflon plates (length: 30.0 cm, width: 20.0 cm, thickness: 2.0 cm) and all components were then threaded through 14 stainless steel screws (length: 34.0 cm, diameter: 7.0 mm) and locked by nuts above stainless steel gasket. The side of each frame was screwed a Teflon joint with which connected rubber tube (6 mm in diameter). The immersion surface area of each electrode in frame cell was 12.0 cm×16.0 cm.

**Experimental procedure:** A clean and dry 5.0 L 4-necked round bottom flask with mechanical stirrer as external container was charged with NaSbF₆ (750 mmol, 194 g, 1.5M.), 1.5 L 2,4,6-collidine, 3.5 L acetonitrile and TMS-enol ether (338 mmol). The mixture was stirred vigorously until a clear, colorless solution was obtained. A peristaltic pump was connected with frame cell, external round bottom flask by rubber tubes (diameter: 8.0 mm) and Teflon tubes (diameter: 6.0 mm) respectively to form a circulatory system. The mixture was then pumped into frame cell with a speed of 500 rpm in the loop by peristaltic pump. The frame cell with a distance of 2.0 cm between each electrode then underwent electrolysis with a constant current of 3.64 A from DC power for 25h and the reaction progress was monitored by GC as well as TLC. Upon completion of the reaction, all mixture was driven into a 30.0 L pail from frame cell and external round bottom flask by peristaltic pump. 6.0 L MeCN was added and circulated for 60 min to wash the frame cell and connecting tubes two times (4 L×2).

Under ice-water bath, 3M HCl (7.2 L) was added into the reaction mixture and stirred vigorously. The mixture was then extracted with MTBE (5 L×3) three times. The combined organic phases were dried over sodium sulfate and concentrated on a rotary evaporator (>120 mbar, 35°C water bath). The crude material obtained as brown oil and was purified by column chromatography.

### Example 1

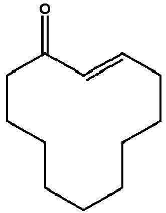

Following the general procedure B for 1.5 hours on (50.8 mg, 0.2 mmol), the titled compound was obtained in 62% yield (22.3 mg, 0.124 mmol) as a white solid. ¹H-NMR (500 MHz, CDCl₃) δ 6.79 (dt, *J* = 15.5, 7.6 Hz, 1H), 6.31 (dt, *J* = 15.7, 1.3 Hz, 1H), 2.60 - 2.39 (m, 2H), 2.26 (tdd, *J* = 7.5, 3.9, 1.2 Hz, 2H), 1.85 - 1.65 (m, 2H), 1.64 - 1.53 (m, 2H), 1.50 - 1.35 (m, 2H), 1.34 - 1.16 (m, 8H). The NMR data were consistent with those previously reported (K. Yamamoto et al., ChemCatChem 2017, 9, 3697-3704).

### Example 2

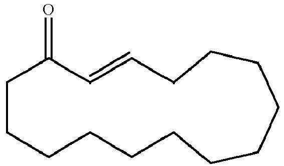

Following the general procedure B for 2 hours on (59.2 mg, 0.2 mmol), the titled compound was obtained in 44% yield (19.5 mg, 0.088 mmol) as a whitish solid. ¹H-NMR (500 MHz, CDCl₃) δ 6.84 (dt, *J* = 15.3, 7.4 Hz, 1H), 6.21 (dt, *J* = 15.7, 1.4 Hz, 1H), 2.55 - 2.49 (m, 2H), 2.33 - 2.25 (m, 2H), 1.75 - 1.66 (m, 2H), 1.64 - 1.52 (m, 2H), 1.40 - 1.16 (m, 16H). The NMR data were consistent with those previously reported (Y. Hisanaga et al., Tetrahedron Lett. 2008, 49, 548-551).

### Example 3

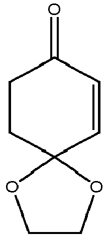

Following the general procedure B for 1.5 hours on (45.6 mg, 0.2 mmol), the titled compound was obtained in 64% yield (19.7 mg, 0.128 mmol) as a yellow solid. ¹H-NMR (400 MHz, CDCl₃) δ 6.60 (dt, *J* = 10.2, 1.0 Hz, 1H), 6.00 (d, *J* = 10.2 Hz, 1H), 4.10 - 3.98 (m, 4H), 2.62 (dd, *J* = 7.1, 6.1 Hz, 2H), 2.19 (ddd, *J* = 7.3, 6.0, 1.0 Hz, 2H). The NMR data were consistent with those previously reported (W. J. Kerr et al., Org. Lett. 2001, 3, 2945-2948).

### Example 4

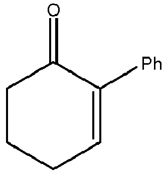

Following the general procedure B for 1.5 hours on (50.8 mg, 0.2 mmol), the titled compound was obtained in 72% yield (24.7 mg, 0.144 mmol) as a white solid. ¹H-NMR (400 MHz, CDCl₃) δ 7.38 - 7.30 (m, 5H), 7.06 (t, *J* = 4.3 Hz, 1H), 2.73 - 2.59 (m, 2H), 2.55 - 2.59 (m, 2H), 2.14 (dq, *J* = 8.2, 6.1 Hz, 2H). The NMR data were consistent with those previously reported (Hayashi, M. et al., Org. Lett. 2012, 14, 154-157).

### Example 5

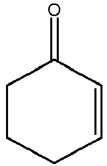

Following the general procedure B for 4 hours on (170 mg, 1 mmol), the titled compound was obtained in 67% yield (64.1 mg, 0.670 mmol) as a yellow oil. ¹H-NMR (500 MHz, CDCl₃) δ 7.00 (dt, *J* = 10.3, 4.1 Hz, 1H), 6.02 (dt, *J* = 10.1, 2.0 Hz, 1H), 2.47 - 2.40 (m, 2H), 2.36 (tdd, *J* = 6.1, 4.1, 2.1 Hz, 2H), 2.07 - 1.98 (m, 2H). The NMR data were consistent with those previously reported (J. Zhang et al., Chem. Commun. 2013, 49, 11662-11664).

### Example 6

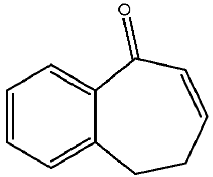

Following the general procedure B for 1.5 hours on (46.4 mg, 0.2 mmol), the titled compound was obtained in 88% yield (27.8 mg, 0.176 mmol) as a colorless oil. ¹H-NMR (500 MHz, CDCl₃) δ 7.78 (d, *J* = 7.8 Hz, 1H), 7.44 (t, *J* = 7.4 Hz, 1H), 7.33 (t, *J* = 7.4 Hz, 1H), 7.22 (d, *J* = 7.3 Hz, 1H), 6.77 (dd, *J* = 11.5, 5.3 Hz, 1H), 6.30 (d, *J* = 12.2 Hz, 1H), 3.12 - 3.07 (m, 2H), 2.62 (s, 2H). The NMR data were consistent with those previously reported (M. Chen et al., Angew. Chem. 2018, 130, 16437-16441).

### Example 7

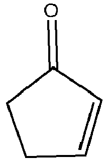

Following the general procedure B for 5 hours on (156 mg, 1 mmol), the titled compound was obtained in 39% yield (32.1 mg, 0.39 mmol) as a colorless oil. ¹H-NMR (500 MHz, CDCl₃) δ7.75 (dt, *J* = 5.5, 2.5 Hz, 1H), 6.24 (dt, *J* = 4.8 Hz, 2.1 Hz, 1H), 2.77 - 2.61 (m, 2H), 2.51 - 2.27 (m, 2H). The NMR data were consistent with those previously reported (J. R. Hwu et al., J. Am. Chem. Soc. 2000, 122, 5899-5900).

### Example 8

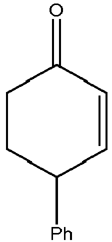

Following the general procedure B for 1.5 hours on (50.8 mg, 0.2 mmol), the titled compound was obtained in 88% yield (30.2 mg, 0.176 mmol) as a colorless oil. ¹H-NMR (500 MHz, CDCl₃) δ 7.39 (dd, *J* = 8.1, 6.9 Hz, 2H), 7.33 - 7.29 (m, 1H), 7.27 - 7.17 (m, 2H), 7.02 (ddd, *J* = 10.1, 2.9, 1.3 Hz, 1H), 6.19 (dd, *J* = 10.2, 2.5 Hz, 1H), 3.75 (ddt, *J* = 9.8, 5.1, 2.7 Hz, 1H), 2.63 - 2.45 (m, 2H), 2.42 - 2.36 (m, 1H), 2.13 - 2.04 (m, 1H). The NMR data were consistent with those previously reported (M. Uyanik et al., J. Am. Chem. Soc. 2009, 131, 251-262).

### Example 9

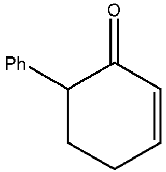

Following the general procedure B for 1.5 hours on (50.8 mg, 0.2 mmol), the titled compound was obtained in 78% yield (26.8 mg, 0.156 mmol) as a colorless oil. ¹H-NMR (400 MHz, CDCl₃) δ 7.54 - 7.29 (m, 2H), 7.22 - 7.15 (m, 2H), 7.06 (ddd, *J* = 10.1, 4.6, 3.6 Hz, 1H), 6.19 (ddd, *J* = 10.1, 2.3, 1.7 Hz, 1H), 3.70 - 3.58 (m, 1H), 2.54 - 2.41 (m, 2H), 2.39 - 2.27 (m, 2H). The NMR data were consistent with those previously reported (S. L. Poe et al., Angew. Chem. Int. Ed. 2011, 50, 4189-4192).

### Example 10

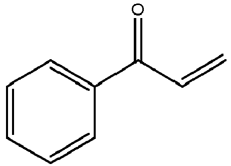

Following the general procedure B for 1.5 hours on (41.2 mg, 0.2 mmol), the titled compound was obtained in 46% yield (12.2 mg, 0.092 mmol) as a colorless oil. ¹H-NMR (400 MHz, CDCl₃) δ 7.96 - 7.93 (m, 2H), 7.60 - 7.56 (m, 1H), 7.50 - 7.46 (m, 2H), 7.17 (dd, *J* = 10.36, 17.12 Hz, 1H), 6.44 (dd, *J* = 1.82, 17.12, 1H), 5.93 (dd, *J* = 1.68, 10.53 Hz, 1H). The NMR data were consistent with those previously reported (F. Verma et al., Adv. Synth. Catal. 2019, 361, 1247-1252).

### Example 11

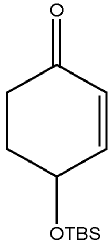

Following the general procedure B for 1.5 hours on (60.0 mg, 0.2 mmol), the titled compound was obtained in 66% yield (29.8 mg, 0.132 mmol) as a colorless oil. ¹H-NMR (400 MHz, CDCl₃) δ 6.85 (dt, *J* = 10.2, 1.8 Hz, 1H), 5.96 (d, *J* = 10.2, 1H), 4.56 (ddt, *J* = 9.2, 4.5, 1.8 Hz, 1H), 2.58 - 263 (m, 1H), 2.35 - 2.41 (m, 1H), 2.20 - 2.27 (m 1H), 1.95 - 2.12 (m, 1H), 0.95 (s, 9H), 0.14 (s, 3H), 0.16 (s, 3H). The NMR data were consistent with those previously reported (P. Bayón et al., J. Org. Chem. 2008, 73, 3486-3491).

### Example 12

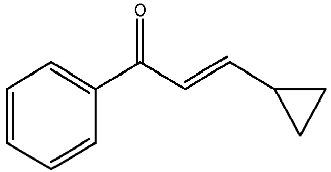

Following the general procedure B for 1.5 hours on (49.2 mg, 0.2 mmol), the titled compound was obtained in 38% yield (13.1 mg, 0.076 mmol) as a colorless oil. ¹H-NMR (400 MHz, CDCl₃) δ8.00-7.92 (m, 2H), 7.60-7.52 (m, 1H), 7.51-7.42 (m, 2H), 7.04 (d, J = 15.1 Hz, 1H), 6.58 (dd, J = 15.1, 10.2 Hz), 1.73 (m, 1H), 1.08-1.00 (m, 2H), 0.79-0.72 (m, 2H).

### Example 13

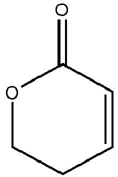

Following the general procedure B for 1.5 hours on (66.4 mg, 0.2 mmol), the titled compound was obtained in 41% yield (8 mg, 0.082 mmol) as a colorless oil. ¹H-NMR (400 MHz, CDCl₃) δ 6.99-6.94 (m, 1H), 6.06 (dt, J = 9.6, 1.6 Hz, 1H), 4.45 (t, J = 6.0 Hz, 2H), 2.48 (tdd, J = 6.4, 4.0, 1.6 Hz, 2H).

### Example 14

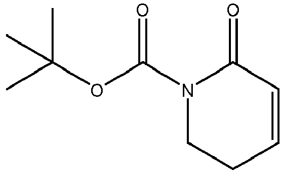

Following the general procedure B for 1.5 hours on (86.2 mg, 0.2 mmol), the titled compound was obtained in 69% yield (27.2 mg, 0.138 mmol) as a colorless oil. ¹H-NMR (400 MHz, CDCl₃) δ 6.35-6.39 (m, 1H), 6.11 (d, J = 9.4, 1H), 3.62 (t, J = 6.9, 2H), 2.27-2.30 (m, 2H), 1.46 (s, 9H).

### Example 15

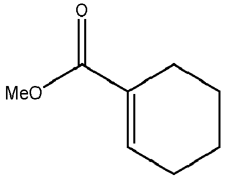

Following the general procedure B for 1.5 hours on (74.8 mg, 0.2 mmol), the titled compound was obtained in 71% yield (20.0 mg, 0.138 mmol) as a colorless oil. ¹H-NMR (400 MHz, CDCl₃) δ 7.01 (m, 1H), 3.75 (s, 3H), 2.24 (m, 2H), 2.27-2.30 (2H, m), 1.64 (m, 9H).

### Example 16

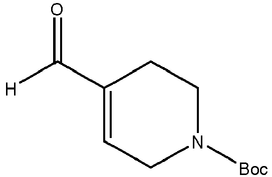

Following the general procedure B for 1.5 hours on (57.1 mg, 0.2 mmol), the titled compound was obtained in 50% yield (21.1 mg, 0.1 mmol) as a colorless oil. ¹H-NMR (400 MHz, CDCl₃) δ 9.49 (s, 1H), 6.72 (br s, 1H), 4.22 - 4.18 (br m, 2H), 3.52 (t, *J* = 5.7 Hz, 2H), 2.34 (br s, 2H), 1.48 (s, 9H).

## Claims

1. Process of α,β-desaturation of a compound comprising a carbonyl moiety comprising the following steps in the following order:
a) preparing a stable enol derivative from the compound comprising a carbonyl moiety;
b) preparing a mixture comprising the enol derivative obtained in step a), a base, an electrolyte and a solvent;
c) electrolyzing the mixture obtained in step b).

2. Process according to claim 1, wherein the compound comprising a carbonyl moiety is selected from ketones, esters, amides, lactones, lactams and aldehydes.

3. Process according to claim 1 or 2, wherein the enol derivative of step a) of the process of the invention is selected from silyl ether enols, enol phosphates and enol esters.

4. Process according to any of claims 1 to 3, wherein step a) is performed in the presence of a base.

5. Process according to any of claims 1 to 4, wherein step a) is performed in the presence of a polar aprotic solvent.

6. Process according to any of claims 1 to 5, wherein the base used in step b) is selected from 2,6-lutidine and 2,4,6-collidine.

7. Process according to any of claims 1 to 5, wherein the amount of base used in step b) is from 10% v/v to 50% v/v with respect to the total volume of the mixture.

8. Process according to any of claims 1 to 6, wherein the electrolyte used in step b) is selected from NaSbF₆, LiClO₄, LiBF₄ and NaOTs.

9. Process according to any of claims 1 to 6, wherein the concentration of electrolyte used in step b) is from 0.01 M to 0.30 M.

10. Process according to any of claims 1 to 7, wherein the solvent used in step b) is a polar aprotic solvent.

11. Process according to any of claims 1 to 10, wherein the step c) is performed at a temperature ranging from 10°C to 50°C.

12. Process according to any of claims 1 to 11, wherein the step c) is performed under a constant current ranging from 1 to 20 mA.
